(19) Europäisches Patentamt
European Patent Office
Office européen des brevets

(11) **EP 4 664 468 A1**

(12) **EUROPEAN PATENT APPLICATION**

(43) Date of publication:
**17.12.2025 Bulletin 2025/51**

(21) Application number: **25180102.3**

(22) Date of filing: **02.06.2025**

(51) International Patent Classification (IPC):
**G16H 20/17** (2018.01)    **G16H 40/63** (2018.01)

(52) Cooperative Patent Classification (CPC):
**G16H 20/17; G16H 40/63**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **13.06.2024 FR 2406266**

(71) Applicant: **Diabeloop**
**38000 Grenoble (FR)**

(72) Inventors:
• **HUNEKER, Erik**
**38120 Saint Egreve (FR)**
• **LACHAL, Sylvain**
**38600 Fontaine (FR)**
• **LOUIS, Maxime**
**38000 Grenoble (FR)**

(74) Representative: **Lannel, Pierre Olivier**
**EINNOVS**
**334, Chemin de la Chapuise**
**69560 Saint-Cyr-sur-le-Rhône (FR)**

(54) **METHOD FOR VALIDATING A CONTROL ALGORITHM**

(57)    A method for validating a control algorithm, the method being implemented by a computer and comprising the steps of receiving a plurality of states (50); receiving a plurality of reference actions (52); receiving a plurality of reference outputs (54); processing the plurality of states (56); computing at least an action difference (58) consisting of computing at least a difference between at least one control action and at least one reference action; evaluating the at least an action difference (60) by computing at least an evaluation score; and validating the control algorithm (62), the control algorithm being validated if at least the evaluation score satisfies an evaluation score criteria.

[Fig. 1]

**Description**

FIELD OF THE INVENTION

The present invention relates to a method and device for validating a control algorithm using states comprising at least a glucose measurement value.

BACKGROUND OF THE INVENTION

In the field of healthcare, and more precisely in the field of treating diabetes, it is known to use control algorithms in order to control the blood glucose, also called glycemia, of a patient inside a safe range also called normoglycemia or euglycemia. The control algorithms send actions, also called control parameters to an insulin pump for example, to inject a quantity of insulin as a function of a glucose measurement value.

Recently, so-called "closed-loop" systems were developed, where a processor is programmed to evaluate a volume rate of insulin to be injected using a control algorithm, based on patient-related data and/or time-based data, such as past and/or present measures of glycemia, and to control the injection of insulin based on this evaluation. In addition, the processor can be programmed to evaluate a volume of insulin to be injected in some special circumstances, in particular meals, and/or physical activity. The quantity can be injected to the patient, subject to the patient's approval. Such systems are also called "hybrid closed-loop" systems because of the necessary declaration by the patient of some of these special circumstances.

An incorrect quantity of insulin to be injected can lead to a concentration of blood glucose in unacceptable intervals, where the patient may be in hypoglycemia and/or hyperglycemia.

Therefore, it is useful to validate a control algorithm before using it in order to increase the security of the patient. Usually, control algorithms are validated during clinical trials.

One main drawback of this method is that it can potentially be dangerous for the patient, therefore said patient must be monitored.

Another drawback is that validating a control algorithm using a clinical trial takes a lot of time.

The invention thus aims to answer at least partially the above presented technical problems.

BRIEF SUMMARY OF THE INVENTION

Thus, the invention relates to a method for validating a control algorithm, the method being implemented by a computer and comprising the steps of:

- receiving a plurality of states, each state comprising at least a glucose measurement value, the glucose measurement value being representative of a measured glucose level of an associated user at an associated time;
- receiving a plurality of reference actions from a reference algorithm, each reference action being associated with a state, and corresponding to a control parameter determined by the reference algorithm based on said associated state;
- receiving a plurality of reference outputs, each reference output corresponding to at least one glucose measurement value and being associated with at least one state;
- processing the plurality of states, each state being processed using the control algorithm to generate a control action associated with said processed state, and corresponding to a control parameter determined by the control algorithm based on said associated state;
- computing at least an action difference, computing at least an action difference consisting of computing at least a difference between at least one control action and at least one reference action;
- evaluating the at least an action difference, evaluating the at least an action difference consisting of computing at least an evaluation score of at least an action difference based on at least a reference output and a predetermined target;
- validating the control algorithm, the control algorithm being validated if at least the evaluation score satisfies an evaluation score criteria.

Such a configuration allows to accurately validate a control algorithm without having to use either a simulator, which is sensitive to bias, or a time consuming clinical trial. Indeed, such a method only needs real data in order to obtain states, reference controls and reference outputs to quickly validate a control algorithm without bias. Indeed, virtual patients simulators are hard to design, often comprise modeling biases - which limits the variability compared to real life and potentially leads to unrealistic simulations - and are associated with potentially large computational costs (such as Ordinary Differential Equation solving by numerical integration).

According to the present invention, a glucose measurement value is a blood glucose measurement value or a value indicative of a blood glucose measurement value such as an interstitial glucose measurement value.

According to the present invention, the predetermined target corresponds to at least one blood glucose value of an associated user.

According to the present invention, a computer is an electronic device that can receive, store, process, and output data according to pre-defined instructions or algorithms such as a smartphone, a server, or a desktop computer for example.

According to an embodiment, the plurality of glucose measurement values are received from a continuous glucose monitoring sensor (CGM) for example.

According to an embodiment, a control parameter is a control parameter configured to control a subcutaneous insulin delivery device configured to deliver exogenous insulin in a subcutaneous tissue of a user in response to a control parameter, in particular continuously infused insulin and/or bolus insulin for example.

According to an embodiment, a state represents the user's state at an associated time.

According to the present invention, each state of the plurality of states also comprises at least one past glucose measurement value representative of a measured glucose level of an associated user at an associated time. Such a configuration allows both the control algorithm and the reference algorithm to generate more accurate actions by estimating a trend, for example, and therefore improve the validating accuracy.

According to the present invention, each state of the plurality of states also comprises at least one past insulin delivery representative of a measured glucose level of an associated user at an associated time. Such a configuration allows both the control algorithm and the reference algorithm to generate more accurate actions by estimating the lasting effect of past insulin deliveries for example and therefore improve the validating accuracy.

According to an embodiment, each state of the plurality of states also comprises at least one past carbohydrate intake representative of a measured glucose level of an associated user at an associated time. Such a configuration allows both the control algorithm and the reference algorithm to be tested on different conditions, such as meal management or rest management for example, and therefore to improve the validating accuracy.

According to an embodiment, a state is a reference output associated with an earlier state. In other words, as the control algorithm is validated according to his capacity to send accurate control parameters allowing maintaining blood glucose of a user close to the target, a state comprising at least a glucose measurement value is a reference output of an earlier state. Indeed, the glucose measurement value associated to a time t+1 is the result/reference output of the reference action taken at a time t based on a state having at least a glucose measurement value associated at a time t.

According to an embodiment, the reference actions and the control actions are positive numbers and are proportional to an amount of insulin to be injected to a user for example.

According to an embodiment, if the control algorithm is validated, the method also comprises a step of sending at least one control parameter to a subcutaneous insulin delivery device configured to deliver exogenous insulin in a subcutaneous tissue of a user in response to a control parameter, in particular continuously infused insulin and/or bolus insulin for example.

According to an embodiment, the reference actions and the control actions can be of any type such as rescue carbs, glucagon, or insulin as long as both reference actions and the control actions are the same type. Such a configuration allows comparing more easily the reference actions and the control actions and therefore to more accurately validate the control algorithm. Furthermore, as rescue carbs, glucagon or insulin have a monotonic impact on the glycemia, it allows to more accurately validate a control algorithm.

According to the present invention, rescue carbs is a recommendation of carbohydrates intakes or a control parameter relative to carbohydrates.

According to an embodiment, the evaluation score criteria can be of any type such as a predetermined threshold for example.

According to an embodiment, the evaluation score criteria corresponds to a threshold assuring that the control algorithm is at least as efficient as the reference algorithm.

According to an embodiment, the evaluation score is computed using a plurality of action differences.

Such a configuration allows to take into account a plurality of situations and therefore more accurately evaluate how the control algorithm would have reacted in particular circumstances represented by the states compared to the reference algorithm.

According to an embodiment, the evaluation score is computed as:

$$\text{evaluation score} = (A + B) / (A + B + C + D);$$

wherein:

A corresponds to proportions of i in [0, ..., N] such that D(i) > 0 and o(i, p+h) > predetermined target;

B corresponds to proportions of i in [0, ..., N] such that D(i) < 0 and o(i, p+h) < predetermined target;

C corresponds to proportions of i in [0, ..., N] such that D(i) > 0 and o(i, p+h) < predetermined target;

D corresponds to proportions of i in [0, ..., N] such that D(i) < 0 and o(i, p+h) > predetermined target;

i corresponds to a sequence of states and associated actions;

N corresponds to a sequence of states and associated actions;

D(i) corresponds to a sum of action differences between indices 0 and p;

p corresponds to a first constant;

h corresponds to a second constant;

o(i, p+h) corresponds to a reference output associated with a sequence of states and associated actions i at a state whose associated time of the glycemia measurement value associated with the state is equal to p+h.

According to the present invention, the term "corresponds to" means "is".

Such a configuration allows creating an objective and unbiased evaluation score as cases falling into the category A are cases for which the end glucose measurement value was above the predetermined target and the control algorithm would have generated a control action corresponding to a greater amount of insulin to be injected: we can assume that the control algorithm was better at dealing with this particular situation, and similarly, mutatis mutandis, for other cases B, C and D.

According to the present invention, p is a first constant corresponding to a number of states, reference actions associated with said states and reference output also associated with said states, small enough so that modifications of insulin delivery during the p number of states, reference actions associated with said states and reference output also associated with said states have not yet sensibly impacted the reference output o(i, p). Such a configuration allows to accurately compute an evaluation score as the insulin has not yet impacted the reference output and therefore said reference output cannot be considered different from a control output. A control output corresponding to at least one glucose measurement value if the associated control action was sent to a subcutaneous insulin delivery device. In other words, having a relatively small p allows, for the user state, to not be modified sensibly between 0 and p under the reference actions, so that assuming it hasn't changed may be valid. As a consequence, the sequences of control actions generated by the control algorithm between 0 and p represent the actions which would have been taken in real life on this user.

According to the present invention, h is a second constant corresponding to a number of states, reference actions associated with said states and reference output also associated with said states, large enough so that modifications of insulin delivery during the p number of states, reference actions associated with said states and reference output also associated with said states, have fully impacted the reference output o(i, p+h). Such a configuration allows to accurately compute an evaluation score as the effect of the control actions taken during the p number of states is fully active at a state in which the associated time of the glycemia measurement value associated with the state is equal to p+h.

According to an embodiment:

A corresponds to proportions of i in [0, ..., N] such that D(i) > 0 and o(i, p+h) > predetermined target + margin high;

B corresponds to proportions of i in [0, ..., N] such that D(i) < 0 and o(i, p+h) < predetermined target - margin low;

C corresponds to proportions of i in [0, ..., N] such that D(i) > 0 and o(i, p+h) < predetermined target + margin high;

D corresponds to proportions of i in [0, ..., N] such that D(i) < 0 and o(i, p+h) > predetermined target - margin low;

Such a configuration allows to artificially reduce the evaluation score and therefore validate only an efficient control algorithm.

Having a margin high different from the margin low allows to artificially reduce the evaluation score more depending on the expected control algorithm efficiency. For example, having a greater margin high allows preventing the validation of a control algorithm promoting only minor improvements of the control output in favor of a control algorithm showing greater improvements, thus avoiding decisions on a control algorithm based only on small glucose measurement value differences due to the variability of continuous glucose monitoring sensors. Having a margin low smaller than the margin high allows reflecting in the margins the smaller distance between dangerous low glucose values leading to hypoglycemia and dangerous high glucose values leading to hyperglycemia. Therefore such a configuration allows validating only an efficient control algorithm.

According to an embodiment, margin high and margin low are variables.

According to the present invention, margin high and margin low vary depending on o(i, p+h), the reference output associated with a sequence of states and associated actions i at a state whose associated time of the glycemia measurement value associated with the state is equal to p+h.

Such a configuration allows validating the control algorithm more safely as it improves the safety of the method by decreasing the variance of the evaluation score, therefore such a configuration allows to more accurately compute an evaluation score and validate only an efficient control algorithm.

According to an embodiment, p is equal to 0.

Such a configuration allows computing an evaluation score based on an action difference of only one control action and reference action. Therefore, the evaluation score will accurately determine the ability of the control algorithm to perform better than the reference algorithm in various cases such as meal bolus estimation for example. According to the present invention, meal bolus estimation means the generation of a control action in response to a carbohydrate intake such as a meal for example.

According to an embodiment, the step of computing at least an action difference consists of computing at least a difference between a sum of a plurality of control actions and a sum of plurality of reference actions.

Such a configuration allows to take into account more actions and therefore allows to more accurately evaluate the at least one action difference at the step of evaluating at least an action difference.

According to an embodiment, the step of computing at least an action difference consists of computing an action difference as:

$$D(i) = (Icont - Iref)/ Irap$$

D(i) corresponds to an action difference of a state i;
Icont corresponds to a sum of a plurality of control actions;
Iref corresponds to a sum of a plurality of reference actions;
Irap corresponds to a normalisation constant.

Such a configuration allows to take into account some particular situations such as D(i) being superior to zero and o(i, p+h) being superior to the predetermined target but with a control parameter strong enough to modify o(i, p+h) as to be below the predetermined target if the control parameter is received by the subcutaneous insulin delivery device instead of the reference parameter for example. Therefore, such a configuration allows to more accurately compute an action difference and therefore more accurately compute an evaluation score and validate only an efficient control algorithm.

According to an embodiment, $Icont = \Sigma j=i-p : i\, F(s(i,j))$, with s(i,j) corresponding to the j-th state of the i-th sequence and F(s(i,j)) corresponding to the j-th control action of the i-th sequence; the control action resulting from the processing of state s(i,j). Such a configuration allows to take into account more actions and therefore allows to more accurately evaluate the at least one action difference at the step of evaluating at least an action difference.

According to an embodiment, $Iref = \Sigma j=i-p : i\, a(i,j)$, with s(i,j) corresponding to the j-th state of the i-th sequence and a(i,j) corresponding to the j-th reference action of the i-th sequence; the reference action resulting from the processing of state s(i,j). Such a configuration allows to take into account more actions and therefore allows to more accurately evaluate the at least one action difference at the step of evaluating at least an action difference.

According to an embodiment in which each state comprises a reference basal rate, Irap varies depending on the value of the reference basal rate. Such a configuration allows to compute a difference action relative to a reference basal rate and therefore compute a customized and meaningful difference action for the associated user. Such a configuration also allows tackling near zero values on the denominator.

According to the present invention, the reference basal rate is a rate of insulin, in units per hour or in fraction of the TDD for example, associated with a user.

According to an embodiment in which each state comprises a reference basal rate and a total daily dose (TDD), if the reference basal rate is superior to a quarter of the TDD and if Iref is superior to zero, Irap = Iref.

According to an embodiment in which each state comprises a reference basal rate and a total daily dose (TDD), if the reference basal rate is superior to a quarter of the TDD and if Iref is equal to zero, Irap = Icont.

According to an embodiment in which each state comprises a reference basal rate and a total daily dose (TDD), if the reference basal rate is inferior or equal to a quarter of the TDD, Irap = (½ TDD)/24.

According to the present invention, the TDD is a total daily dose of insulin associated with a user and theoretically needed by the associated user to maintain glucose values in normoglycemia.

According to an embodiment, the step of evaluating the at least an action difference consists of computing an evaluation score of the action difference based on a difference between one reference output and the predetermined target.

Such a configuration allows for improved validation accuracy of the control algorithm by comparing action differences with a reference output and a predefined target. This improvement is achieved by using an evaluation score based on the difference between the reference output and the predetermined target, which provides a more precise measure of the control algorithm's efficiency. The comparison with a reference output ensures that the method can effectively identify discrepancies, leading to a more accurate validation method.

Such a configuration allows for easier identification of efficiency gaps between the control algorithm being validated and a reference algorithm through the evaluation of action differences. This ease of identification is obtained by comparing the predetermined target with the reference output, which highlights discrepancies in the algorithm's efficiency. By pinpointing these discrepancies, the method can effectively identify areas for improvement and facilitate the optimization of the control algorithm under validation.

According to an embodiment, D(g) represents the difference between one reference output and the predetermined target.

According to an embodiment, the step of evaluating the at least an action difference consists of computing an evaluation score of the action difference based on both D(i) and D(g). Such a configuration allows to more accurately compute an evaluation score as the said evaluation score depends on both the result through the reference output in view of the expectation through the predetermined target and the control action in view of the reference action. Indeed, if the reference action results in a reference output only slightly under or above the predetermined target, a control parameter too far from the reference control would not deserve a high evaluation score.

According to an embodiment, the plurality of control actions and the plurality of reference actions are associated with the same plurality of states and wherein the at least a glucose measurement value of the same plurality of states is associated with time in at least one continuous time interval.

According to the present invention, a continuous time interval is continuous relatively to the associated time of the at least a glucose measurement value. **In** other words, all the states comprising at least a glucose measurement value associated with a time within the continuous time interval are included in the same plurality of states. A past glucose measurement value, while having an associated time is not considered as a glucose measurement value in this particular case as it refers to a glucose measurement value made in the past.

According to an embodiment, the plurality of control actions and the plurality of reference actions are associated with the same plurality of states and wherein the at least a glucose measurement value of the same plurality of states are associated with time in a plurality of continuous time intervals related to at least a particular period of a day.

According to the present invention, a particular period of the day could be of any type such as postprandial period, meal period, physical activity period or sleep period for example.

Such a configuration allows the method to validate the control algorithm specifically for a period of the day. Therefore, a user could use a plurality of control algorithms, each control algorithm being validated for each particular period of the day. Such a configuration allows to greatly improve blood glucose regulation of a user.

According to an embodiment, the step of computing at least an action difference consists of computing at least a difference between at least one control action and at least one reference action as follows:

$$D(i) = \sum(k = j \ldots p)(F(s(i,j)) - a(i,j))$$

s(i,j) corresponds to the j-th state of the i-th sequence;
a(i,j) corresponds to the j-th reference action of the i-th sequence; the reference action resulting from the processing of state s(i,j); and
F(s(i,j)) corresponds to the j-th control action of the i-th sequence; the control action resulting from the processing of state s(i,j).

According to an embodiment, the step of validating the control algorithm consists of validating the control algorithm if the evaluation score satisfies an evaluation score criteria and if at least one of a first worst cases score and a second worst cases score satisfies a worst cases score criteria, wherein the first worst cases score is computed as:

first worst cases score = average(i such as D(i) > 0 and o(i, p+h) < target); and wherein
the second worst cases score is computed as:
second worst cases score = average(i such as D(i) < 0 and o(i, p+h) > target).
i corresponds to a number of states;
D(i) corresponds to a sum of an action differences between indices 0 and p;
o(i, p+h) corresponds to a reference output associated with a sequence of states and associated actions i at a state whose associated time of the glycemia measurement value associated with the state is equal to p+h.

Such a configuration allows the method to only validate a control algorithm that has an acceptable first worst cases score or second worst cases score and therefore furthermore improves the security of the method.

According to an embodiment, the step of validating the control algorithm consists of validating the control algorithm if the evaluation score satisfies an evaluation score criteria and if the first worst cases score satisfies the worst cases score criteria. Such a configuration allows the method to only validate a control algorithm that has an acceptable first worst case score corresponding to a glucose value inferior to the target such as an hypoglycemia for example and therefore furthermore improves the security of the method.

According to an embodiment, the step of validating the control algorithm consists of validating the control algorithm if the evaluation score satisfies an evaluation score criteria and if the first worst cases score and the second worst cases score both satisfy the worst cases score criteria. Such a configuration allows the method to only validate a control algorithm that has an acceptable first worst cases score and second worst cases score and therefore furthermore improves the security of the method.

According to an embodiment, the first worst cases score and the second worst cases score can be of any type such as a predetermined threshold for example.

According to an embodiment, the first worst cases score and the second worst cases score correspond to thresholds assuring that the control algorithm never generates, during the step of processing the plurality of states, a worst control parameter than the reference parameter while said reference parameter leads the reference outputs outside a safe range. A safe range can be of any type such as a range corresponding to normoglycemia for example.

The invention also relates to an automated closed-loop blood glucose control system for the controlled delivery of insulin to a user comprising:

a continuous glucose monitoring sensor configured to provide a plurality of glucose measurement values representative of a measured glucose level of the user at an associated plurality of measurement times;
a subcutaneous insulin delivery device configured to deliver exogenous insulin in a subcutaneous tissue of a user in response to a control parameter;
a controller programmed to receive the glucose measurement values and provide a control parameter to the subcutaneous insulin delivery device;
wherein the controller is programmed to determine the control parameter using a validated control algorithm according to the embodiments above described.

The various non-incompatible aspects defined above can be combined.

BRIEF DESCRIPTION OF THE DRAWINGS

Embodiments of the invention will be described below with reference to the drawings, described briefly below:

- [Fig. 1] shows a method for validating a control algorithm according to one embodiment of the invention; and
- [Fig. 2] shows an automated closed-loop blood glucose control system according to one embodiment of the invention.

DETAILED DESCRIPTION OF THE INVENTION

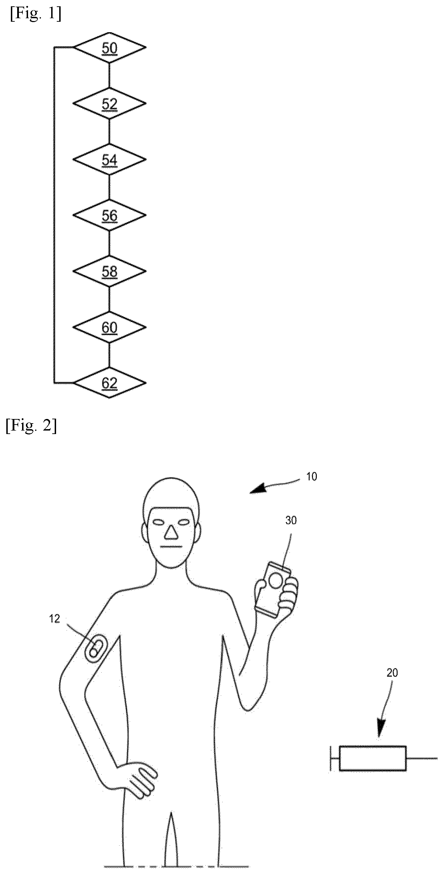

Figure 1 represents a method for validating a control algorithm, the method being implemented by a computer and comprising a step of receiving a plurality of states 50, each state comprising at least a glucose measurement value, the glucose measurement value being representative of a measured glucose level of an associated user at an associated time. According to the present invention, a glucose measurement value is a blood glucose measurement value or a value indicative of a blood glucose measurement value such as an interstitial glucose measurement value. According to the present invention, a computer is an electronic device that can receive, store, process, and output data according to predefined instructions or algorithms such as a smartphone, a server, or a desktop computer for example. The plurality of glucose measurement values are received from a continuous glucose monitoring sensor (CGM) for example.

A state represents the user's state at an associated time. Each state of the plurality of states also comprises at least one past glucose measurement value representative of a measured glucose level of an associated user at an associated time. Such a configuration allows both the control algorithm and the reference algorithm to generate more accurate actions by estimating a trend, for example, and therefore improve the validation accuracy. Each state of the plurality of states also comprises at least one past insulin delivery of an associated user at an associated time. Such a configuration allows both the control algorithm and the reference algorithm to generate more accurate actions by estimating the lasting effect of past insulin deliveries for example and therefore improve the validation accuracy. Each state of the plurality of states also comprises at least one past carbohydrate intake representative of a measured glucose level of an associated user at an associated time. Such a configuration allows both the control algorithm and the reference algorithm to be tested on different conditions, such as meal management or rest management for example, and therefore to improve the validation accuracy. Therefore, a state is also a reference output associated with an earlier state. In other words, as the control algorithm is validated according to his capacity to send accurate control parameters allowing maintaining blood glucose of a user close to the target, a state comprising at least a glucose measurement value is a reference output of an earlier state. Indeed, the glucose measurement value associated to a time t+1 is the result/reference output of a reference action taken at a time t based on a state having at least a glucose measurement value associated at a time t.

The method comprises a step of receiving a plurality of reference actions 52 from a reference algorithm, each reference action being associated with a state so that a particular action is associated with the particular state representative of said action's consequences, and corresponding to a control parameter determined by the reference algorithm based on said associated state. A control parameter is a control parameter configured to control a subcutaneous insulin delivery device configured to deliver exogenous insulin in a subcutaneous tissue of a user in response to a control parameter, in particular continuously infused insulin and/or bolus insulin.

The method comprises a step of receiving a plurality of reference outputs 54, each reference output corresponding to at least one glucose measurement value and being associated with at least one state.

The method comprises a step of processing the plurality of states 56, each state being processed using the control algorithm to generate a control action associated with said processed state, and corresponding to a control parameter determined by the control algorithm based on said associated state. The reference actions and the control actions are positive numbers and are proportional to an amount of insulin to be injected to a user for example. The reference actions and the control actions can be of any type such as rescue carbs, glucagon, or insulin as long as both reference actions and the control actions are the same type. Such a configuration allows comparing more easily the reference actions and the control actions and therefore to more accurately validate the control algorithm. Furthermore, as rescue carbs, glucagon or insulin have a monotonic impact on the glycemia, it allows to more accurately validate a control algorithm. For example, a monotonic impact is when more insulin is injected to a user, the user's glycemia will decrease, unless carbohydrates are ingested. According to the present invention, rescue carbs is a recommendation of carbohydrates intakes or a control parameter relative to carbohydrates.

The method comprises a step of computing at least an action difference 58, computing at least an action difference 58 consisting of computing at least a difference between at least one control action and at least one reference action. Furthermore, the step of computing at least an action difference 58 consists of computing at least a difference between a sum of a plurality of control actions and a sum of plurality of reference actions. Such a configuration allows to take into account more actions and therefore allows to more accurately evaluate the at least one action difference at the step of evaluating at least an action difference.

More particularly, the step of computing at least an action difference 58 consists of computing an action difference as:

$$D(i) = (Icont - Iref)/ Irap$$

D(i) corresponds to an action difference of a state i;
Icont corresponds to a sum of a plurality of control actions;
Iref corresponds to a sum of a plurality of reference actions;
Irap corresponds to a normalisation constant. Irap is used to convert absolute insulin amounts into 'relative amount' compared to the user basic insulin needs, so that each user contributes as much as any other user to the fine tuning.

Such a configuration allows to take into account some particular situations such as D(i) being superior to zero and o(i, p+h) being superior to the predetermined target but with a control parameter strong enough to modify o(i, p+h) as to be below the predetermined target if the control parameter is received by the subcutaneous insulin delivery device instead of the reference parameter for example. Therefore, such a configuration allows to more accurately compute an action difference therefore such a configuration allows to more accurately compute an evaluation score and validate only an efficient control algorithm.

Iref = Σj=i-p : i a(i,j), with s(i,j) corresponding to the j-th state of the i-th sequence and a(i,j) corresponding to the j-th reference action of the i-th sequence; the reference action resulting from the processing of state s(i,j). Such a configuration allows to take into account more actions and therefore allows to more accurately evaluate the at least one action difference at the step of evaluating at least an action difference.

Icont = Σj=i-p : i F(s(i,j)), with s(i,j) corresponding to the j-th state of the i-th sequence and F(s(i,j)) corresponding to the j-th control action of the i-th sequence; the control action resulting from the processing of state s(i,j). Such a configuration allows to take into account more actions and therefore allows to more accurately evaluate the at least one action difference at the step of evaluating at least an action difference. Iref = Σj=i-p : i a(i,j), with s(i,j) corresponding to the j-th state of the i-th sequence and a(i,j) corresponding to the j-th reference action of the i-th sequence; the reference action resulting from the processing of state s(i,j). Such a configuration allows to take into account more actions and therefore allows to more accurately evaluate the at least one action difference at the step of evaluating at least an action difference.

Each state comprises a reference basal rate and a total daily dose (TDD) and Irap varies depending on the value of the reference basal rate. Such a configuration allows computing a difference action relative to a reference basal rate and therefore compute a customised and meaningful difference action for the associated user. Such a configuration allows obtaining a normalised metric more independent of the user. The reference basal rate is a rate of insulin, in units per hour or in fraction of the TDD for example, associated with a user. if the reference basal rate is superior to a quarter of the TDD and if Iref is superior to zero, Irap = Iref. If the reference basal rate is superior to a quarter of the TDD and if Iref is equal to zero, Irap = Icont. If the reference basal rate is inferior or equal to a quarter of the TDD, Irap = (½ TDD)/24. According to the present invention, the TDD is a total daily dose of insulin associated with a user and theoretically needed by the associated user to maintain glucose values in normoglycemia. Such a configuration also allows tackling near zero values on the denominator.

According to another embodiment, the step of computing at least an action difference 58 consists of computing at least a difference between at least one control action and at least one reference action as follows:

$$D(i) = \sum(k = j \ldots p)(F(s(i,j)) - a(i,j))$$

s(i,j) corresponds to the j-th state of the i-th sequence;
a(i,j) corresponds to the j-th reference action of the i-th sequence; the reference action resulting from the processing of state s(i,j); and
F(s(i,j)) corresponds to the j-th control action of the i-th sequence; the control action resulting from the processing of state s(i,j).

The method comprises a step of evaluating the at least an action difference 60, evaluating the at least an action difference 60 consisting of computing at least an evaluation score of at least an action difference based on at least a reference output and a predetermined target. According to the present invention, the predetermined target corresponds to at least one blood glucose value of an associated user. The step of evaluating the at least an action difference 60 consists of computing an evaluation score of the action difference based on a difference between one reference output and the predetermined target. Such a configuration allows for improved validation accuracy of the control algorithm by comparing action differences with a reference output and a predefined target. This improvement is achieved by using an evaluation score based on the difference between the reference output and the predetermined target, which provides a more precise measure of the control algorithm's efficiency. The comparison with a reference output ensures that the method can effectively identify discrepancies, leading to a more accurate validation method. Such a configuration also allows for easier identification of efficiency gaps between the control algorithm being validated and a reference algorithm through the evaluation of action differences. This ease of identification is obtained by comparing the predetermined target with the reference output, which highlights discrepancies in the algorithm's efficiency. By pinpointing these discrepancies, the method can effectively identify areas for improvement and facilitate the optimization of the control algorithm under validation.

The step of evaluating the at least an action difference 60 consists of computing an evaluation score of the action difference based on both D(i) and D(g) where D(g) represents the difference between one reference output and the predetermined target. Such a configuration allows to more accurately compute an evaluation score as the said evaluation score depends on both the result through the reference output in view of the expectation through the predetermined target and the control action in view of the reference action. Indeed, if the reference action results in a reference output only slightly under or above the predetermined target, a control parameter too far from the reference control would not deserve a high evaluation score.

The method comprises a step of validating the control algorithm 62, the control algorithm being validated if at least the evaluation score satisfies an evaluation score criteria. The evaluation score criteria corresponds to a threshold assuring that the control algorithm is at least as efficient as the reference algorithm. Such a configuration allows to accurately validate a control algorithm without having to use either a simulator, which is sensitive to bias, or a time consuming clinical trial. Indeed, such a method only needs real data in order to obtain states, reference control and reference output to quickly validate a control algorithm without bias. Indeed, virtual patients simulators are hard to design, often comprise modeling biases - which limits the variability compared to real life and potentially leads to unrealistic simulations - and are associated with potentially large computational costs (such as Ordinary Differential Equation solving by numerical integration).

The evaluation score is computed using a plurality of action differences. Such a configuration allows to take into account a plurality of situations and therefore more accurately evaluate how the control algorithm would have reacted in particular circumstances represented by the states compared to the reference algorithm.

According to a certain embodiment, the evaluation score is computed as:

$$\text{evaluation score} = (A + B) / (A + B + C + D);$$

wherein:

A corresponds to proportions of i in [0, ..., N] such that D(i) > 0 and o(i, p+h) > predetermined target;
B corresponds to proportions of i in [0, ..., N] such that D(i) < 0 and o(i, p+h) < predetermined target;
C corresponds to proportions of i in [0, ..., N] such that D(i) > 0 and o(i, p+h) < predetermined target;
D corresponds to proportions of i in [0, ..., N] such that D(i) < 0 and o(i, p+h) > predetermined target;
i corresponds to a sequence of states and associated actions;
N corresponds to a sequence of states and associated actions;
D(i) corresponds to a sum of action differences between indices 0 and p;
p corresponds to a first constant;
h corresponds to a second constant;
o(i, p+h) corresponds to a reference output associated with a sequence of states and associated actions i at a state whose associated time of the glycemia measurement value associated with the state is equal to p+h.

Such a configuration allows creating an objective and unbiased evaluation score as cases falling into the category A are cases for which the end glucose measurement value was above the predetermined target and the control algorithm would have generated a control action corresponding to a greater amount of insulin to be injected: we can assume that the control algorithm was better at dealing with this particular situation, and similarly, mutatis mutandis, for other cases B, C and D.

p is a first constant corresponding to a number of states, reference actions associated with said states and reference outputs also associated with said states, small enough so that modifications of insulin delivery during the p number of states, reference actions associated with said states and reference outputs also associated with said states have not yet sensibly impacted the reference output o(i, p). Such a configuration allows to accurately compute an evaluation score as the insulin has not yet impacted the reference output and therefore said reference output cannot be considered different from a control output. A control output corresponding to at least one glucose measurement value if the associated control action was sent to a subcutaneous insulin delivery device. In other words, having a relatively small p allows, for the user state, to not be modified sensibly under the control actions compared to the reference actions. As a consequence, the sequences of control actions generated by the control algorithm between 0 and p are very close to the actions which would have been taken in real life on this user. h is a second constant corresponding to a number of states, reference actions associated with said states and reference outputs also associated with said states, large enough so that modifications of insulin delivery during the p number of states, reference actions associated with said states and reference outputs also associated with said states, have fully impacted the reference output o(i, p+h). Such a configuration allows to accurately compute an evaluation score as the effect of the control actions taken during the p number of states is fully active at a state in which the associated time of the glycemia measurement value associated with the state is equal to p+h.

According to another certain embodiment, A corresponds to proportions of i in [0, ..., N] such that D(i) > 0 and o(i, p+h) > predetermined target + margin high;

B corresponds to proportions of i in [0, ..., N] such that D(i) < 0 and o(i, p+h) < predetermined target - margin low;
C corresponds to proportions of i in [0, ..., N] such that D(i) > 0 and o(i, p+h) < predetermined target + margin high;
D corresponds to proportions of i in [0, ..., N] such that D(i) < 0 and o(i, p+h) > predetermined target - margin low;

Such a configuration allows to artificially reduce the evaluation score and therefore validate only an efficient control algorithm.

According to any of the certain embodiments, margin high and margin low are variables and vary depending on o(i, p+h), the reference output associated with a sequence of states and associated actions i at a state whose associated time of the glycemia measurement value associated with the state is equal to p+h. Such a configuration allows to validate the control algorithm more safely as it improves the safety of the method by decreasing the variance of the evaluation score, therefore such a configuration allows to more accurately compute an evaluation score and validate only an efficient control algorithm.

According to any of the certain embodiments, p is equal to 0. Such a configuration allows to compute an evaluation score based on an action difference of only one control action and reference action. Therefore, the evaluation score will accurately determine the ability of the control algorithm to perform better than the reference algorithm in various cases such as meal bolus estimation for example. According to the present invention, meal bolus estimation means the generation of a control action in response to a carbohydrate intake such as a meal for example.

The plurality of control actions and the plurality of reference actions are associated with the same plurality of states and the at least a glucose measurement value of the same plurality of states is associated with time in at least one continuous time interval. According to the present invention, a continuous time interval is continuous relatively to the associated time of the at least a glucose measurement value. In other words, all the states comprising at least a glucose measurement value associated with a time within the continuous time interval are included in the same plurality of states. A past glucose measurement value, while having an associated time is not considered as a glucose measurement value in this particular case as it refers to a glucose measurement value made in the past.

The plurality of control actions and the plurality of reference actions are associated with the same plurality of states and the at least a glucose measurement value of the same plurality of states are associated with time in a plurality of continuous time intervals related to at least a particular period of a day. According to the present invention, a particular period of the day could be of any type such as postprandial period, meal period, physical activity period or sleep period for example. Such a configuration allows the method to validate the control algorithm specifically for a period of the day. Therefore, a user could use a plurality of control algorithms, each control algorithm being validated for each particular period of the day. Such a configuration allows to greatly improve blood glucose regulation of a user.

According to a certain embodiment, the step of validating the control algorithm 62 consists of validating the control algorithm if the evaluation score satisfies an evaluation score criteria and if at least one of a first worst cases score and a second worst cases score satisfies a worst cases score criteria, wherein the first worst cases score is computed as:

first worst cases score = average(i such as D(i) > 0 and o(i, p+h) < target); and whereinthe second worst cases score is computed as:

$$\text{second worst cases score} = \text{average}(i \text{ such as } D(i) < 0 \text{ and } o(i, p+h) > \text{target}).$$

i corresponds to a number of states;
D(i) corresponds to a sum of an action differences between indices 0 and p;
o(i, p+h) corresponds to a reference output associated with a sequence of states and associated actions i at a state whose associated time of the glycemia measurement value associated with the state is equal to p+h.

Such a configuration allows the method to only validate a control algorithm that has an acceptable first worst cases score or second worst cases score and therefore furthermore improves the security of the method.

The step of validating the control algorithm 62 can also consist of validating the control algorithm if the evaluation score satisfies an evaluation score criteria and if the first worst cases score satisfies the worst cases score criteria. Such a configuration allows the method to only validate a control algorithm that has an acceptable first worst cases score corresponding to a glucose value inferior to the target such as an hypoglycemia for example and therefore furthermore improves the security of the method.

The step of validating the control algorithm 62 can also consist of validating the control algorithm if the evaluation score satisfies an evaluation score criteria and if the first worst cases score and the second worst cases score both satisfy the worst cases score criteria. Such a configuration allows the method to only validate a control algorithm that has an acceptable first worst cases score and second worst cases score and therefore furthermore improves the security of the method. the first worst cases score and the second worst cases score can be of any type such as a predetermined threshold for example. More particularly, the first worst cases score and the second worst cases score correspond to thresholds assuring that the control algorithm never generates, during the step of processing the plurality of states 56, a worst control parameter than the reference parameter while said reference parameter leads the reference outputs outside a safe range. A safe range can be of any type such as a range corresponding to normoglycemia for example.

According to an embodiment, the step of validating the control algorithm 62 consists of validating the control algorithm if the evaluation score satisfies an evaluation score criteria and if a case score satisfies a case criteria. The case score represents an evaluation of the number of good, bad and worse control actions by considering states where the control action recommended rescue carbs and the reference action did not. A good case corresponds to a reference output showing hypoglycemia. A bad case corresponds to a reference output showing a normoglycemia. A worse case corresponds to a reference output showing an hyperglycemia. Such a configuration allows evaluating the control algorithm based on some specific cases and therefore increases the efficiency of the method. The same reasoning can be applied considering boluses instead of rescue carbs. The same reasoning can be applied considering insulin cutoff such as basal cutoff for example. The considered cases are cases where reference actions correspond to a smaller cutoff than control actions.

If the control algorithm is validated, the method also comprises a step of sending at least one control parameter to a subcutaneous insulin delivery device configured to deliver exogenous insulin in a subcutaneous tissue of a user in response to a control parameter, in particular continuously infused insulin and/or bolus insulin.

According to an embodiment in which the control algorithm comprises a design parameter influencing the control action generated at the step of processing a plurality of states, the method for validating a control algorithm also comprises a step of optimising the design parameter by using any numerical optimisation algorithm on the function:

$$F(W) = (A + B) / (A + B + C + D)$$

W corresponds to the design parameter.

Such an embodiment allows optimisation and therefore improves the control algorithm.

The numerical optimisation algorithm can be of any type such as convex, differentiable, constrained, line search, gradient descent, Hessian matrix or stochastic optimisation for example.

The invention also relates to an automated closed-loop blood glucose control system 10 for the controlled delivery of insulin to a user comprising:

- a continuous glucose monitoring sensor 12 configured to provide a plurality of glucose measurement values representative of a measured glucose level of the user at an associated plurality of measurement times;
- a subcutaneous insulin delivery device 20 configured to deliver exogenous insulin in a subcutaneous tissue of a user in response to a control parameter;
- a controller 30 programmed to receive the glucose measurement values and provide a control parameter to the subcutaneous insulin delivery device;
- wherein the controller is programmed to determine the control parameter using a validated control algorithm according to what was described above.

Obviously, the different embodiments and technical effects described above can be applied to the blood glucose control system.

While exemplary embodiments of the invention has been described, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiments disclosed for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

**Claims**

1.  A method for validating a control algorithm, the method being implemented by a computer and comprising the steps of:

     • receiving a plurality of states (50), each state comprising at least a glucose measurement value, the glucose measurement value being representative of a measured glucose level of an associated user at an associated time;
     • receiving a plurality of reference actions (52) from a reference algorithm, each reference action being associated with a state, and corresponding to a control parameter determined by the reference algorithm based on said associated state;
     • receiving a plurality of reference outputs (54), each reference output corresponding to at least one glucose measurement value and being associated with at least one state;
     • processing the plurality of states (56), each state being processed using the control algorithm to generate a control action associated with said processed state, and corresponding to a control parameter determined by the control algorithm based on said associated state;
     • computing at least an action difference (58), computing at least an action difference consisting of computing at least a difference between at least one control action and at least one reference action;
     • evaluating the at least an action difference (60), evaluating the at least an action difference consisting of computing at least an evaluation score of at least an action difference based on at least a reference output and a predetermined target;
     • validating the control algorithm (62), the control algorithm being validated if at least the evaluation score satisfies an evaluation score criteria.

2. Method according to claim 1, wherein the evaluation score is computed using a plurality of action differences.

3. Method according to claim 2, wherein the evaluation score is computed as:

$$\text{evaluation score} = (A + B) / (A + B + C + D);$$

wherein:

A corresponds to proportions of i in [0, ..., N] such that D(i) > 0 and o(i, p+h) > predetermined target;
B corresponds to proportions of i in [0, ..., N] such that D(i) < 0 and o(i, p+h) < predetermined target;
C corresponds to proportions of i in [0, ..., N] such that D(i) > 0 and o(i, p+h) < predetermined target;
D corresponds to proportions of i in [0, ..., N] such that D(i) < 0 and o(i, p+h) > predetermined target;
i corresponds to a sequence of states and associated actions;
N corresponds to a sequence of states and associated actions;
D(i) corresponds to a sum of action differences between indices 0 and p;
p corresponds to a first constant;
h corresponds to a second constant;
o(i, p+h) corresponds to a reference output associated with a sequence of states and associated actions i at a state whose associated time of the glycemia measurement value associated with the state is equal to p+h.

4. Method according to claim 3, wherein:

A corresponds to proportions of i in [0, ..., N] such that D(i) > 0 and o(i, p+h) > predetermined target + margin high;
B corresponds to proportions of i in [0, ..., N] such that D(i) < 0 and o(i, p+h) < predetermined target - margin low;
C corresponds to proportions of i in [0, ..., N] such that D(i) > 0 and o(i, p+h) < predetermined target + margin high;
D corresponds to proportions of i in [0, ..., N] such that D(i) < 0 and o(i, p+h) > predetermined target - margin low.

5. Method according to claim 4, wherein margin high and margin low are variables.

6. Method according to any of the claims 3 to 5, wherein p is equal to 0.

7. Method according to any of the claims 1 to 6, wherein the step of computing at least an action difference (58) consists of computing at least a difference between a sum of a plurality of control actions and a sum of plurality of reference actions.

8. Method according to claim 7, wherein the step of computing at least an action difference (58) consists of computing an action difference as:

$$D(i) = (I_{cont} - I_{ref}) / I_{rap}$$

D(i) corresponds to an action difference of a state i;
$I_{cont}$ corresponds to a sum of a plurality of control actions;
$I_{ref}$ corresponds to a sum of a plurality of reference actions;
$I_{rap}$ Irap corresponds to a normalisation constant.

9. Method according to claim 8, wherein the step of evaluating the at least an action difference (60) consists of computing an evaluation score of the action difference based on a difference between one reference output and the predetermined target.

10. Method according to any of the claims 7 to 9, wherein the plurality of control actions and the plurality of reference actions are associated with the same plurality of states and wherein the at least a glucose measurement value of the same plurality of states is associated with time in at least one continuous time interval.

11. Method according to claim 10, wherein the plurality of control actions and the plurality of reference actions are associated with the same plurality of states and wherein the at least a glucose measurement value of the same plurality of states are associated with time in a plurality of continuous time intervals related to at least a particular period of a day.

12. Method according to any of the claims 1 to 11, wherein the step of computing at least an action difference (58) consists of computing at least a difference between at least one control action and at least one reference action as follows:

$$D(i) = \sum(k = j \ldots p)(F(s(i,j)) - a(i,j))$$

s(i,j) corresponds to the j-th state of the i-th sequence;
a(i,j) corresponds to the j-th reference action of the i-th sequence; the reference action resulting from the processing of state s(i,j); and
F(s(i,j)) corresponds to the j-th control action of the i-th sequence; the control action resulting from the processing of state s(i,j).

13. Method according to any of the claims 1 to 12, wherein the step of validating the control algorithm (62) consists of validating the control algorithm if the evaluation score satisfies an evaluation score criteria and if at least one of a first worst cases score and a second worst cases score satisfies a worst cases score criteria, wherein the first worst cases score is computed as:

first worst cases score = average(i such as D(i) > 0 and o(i, p+h) < target) ; and
the second worst cases score is computed as:

second worst cases score = average(i such as D(i) < 0 and o(i, p+h) > target).
i corresponds to a number of states;
D(i) corresponds to a sum of an action differences between indices 0 and p;
o(i, p+h) corresponds to a reference output associated with a sequence of states and associated actions i at a state whose associated time of the glycemia measurement value associated with the state is equal to p+h.

14. An automated closed-loop blood glucose control system (10) for the controlled delivery of insulin to a user comprising:

• a continuous glucose monitoring sensor (12) configured to provide a plurality of glucose measurement values representative of a measured glucose level of the user at an associated plurality of measurement times;
• a subcutaneous insulin delivery device (20) configured to deliver exogenous insulin in a subcutaneous tissue of a user in response to a control parameter;
• a controller (30) programmed to receive the glucose measurement values and provide a control parameter to the subcutaneous insulin delivery device;

wherein the controller is programmed to determine the control parameter using a validated control algorithm according to any of the claims 1 to 13.

[Fig. 1]

[Fig. 2]

Europäisches
Patentamt

European
Patent Office

Office européen
des brevets

# EUROPEAN SEARCH REPORT

Application Number

EP 25 18 0102

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | US 2024/024576 A1 (BRETON MARC D [US] ET AL) 25 January 2024 (2024-01-25)<br>* abstract *<br>* paragraphs [0002], [0062], [0072], [0075], [0077]; claim 1 *<br>----- | 1,2,<br>7-12,14<br>3-6,13 | INV.<br>G16H20/17<br>G16H40/63 |

TECHNICAL FIELDS
SEARCHED      (IPC)

G16H

The present search report has been drawn up for all claims

| Place of search | Date of completion of the search | Examiner |
|---|---|---|
| Munich | 8 October 2025 | Schmitt, Constanze |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

\.......................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 18 0102

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2025

| Patent document cited in search report | Publication date | Patent family member(s) | | Publication date |
|---|---|---|---|---|
| US 2024024576 A1 | 25-01-2024 | AU | 2021360836 A1 | 15-06-2023 |
| | | CA | 3195456 A1 | 21-04-2022 |
| | | CN | 116670774 A | 29-08-2023 |
| | | EP | 4228498 A1 | 23-08-2023 |
| | | JP | 2023551633 A | 12-12-2023 |
| | | KR | 20230107245 A | 14-07-2023 |
| | | US | 2024024576 A1 | 25-01-2024 |
| | | WO | 2022081788 A1 | 21-04-2022 |

EPO FORM P0459

For more details about this annex : see Official Journal of the European Patent Office, No. 12/82